(19) Europäisches Patentamt
European Patent Office
Office européen des brevets

(11) **EP 4 534 521 A1**

(12) **EUROPEAN PATENT APPLICATION**
published in accordance with Art. 153(4) EPC

(43) Date of publication:
**09.04.2025  Bulletin 2025/15**

(21) Application number: **23816139.2**

(22) Date of filing: **01.06.2023**

(51) International Patent Classification (IPC):
*C07C 47/04* (2006.01)    *C07C 309/04* (2006.01)
*C07D 323/06* (2006.01)

(52) Cooperative Patent Classification (CPC):
**C07C 47/04; C07C 309/04; C07D 323/06**

(86) International application number:
**PCT/JP2023/020399**

(87) International publication number:
**WO 2023/234382 (07.12.2023 Gazette 2023/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **03.06.2022   JP 2022091017**

(71) Applicant: **MITSUBISHI GAS CHEMICAL
COMPANY, INC.
Chiyoda-ku
Tokyo 100-8324 (JP)**

(72) Inventor: **OKADA Takuya
Yokkaichi-shi, Mie 510-0886 (JP)**

(74) Representative: **Müller-Boré & Partner
Patentanwälte PartG mbB
Friedenheimer Brücke 21
80639 München (DE)**

(54) **COMPOSITION, AND METHOD FOR PRODUCING TRIOXANE USING SAME**

(57)    Provided is a means capable of continuously producing trioxane in high yield.

A composition containing (A) 58.5 to 65.5 mass% of formaldehyde, (B) 0.7 to 4.5 mass% of methanesulfonic acid; and (C) less than 0.007 mass% of a metal salt, wherein a total content of the formaldehyde (A) and the methanesulfonic acid (B) is less than 68 mass%, and the metal salt (C) contains at least one selected from a group consisting of a nitrate, a nitrite, and a persulfate.

## Description

Technical Field

The present invention relates to a composition and a method for producing trioxane using the same.

Background Art

Polyoxymethylene (POM) is excellent in strength, elastic modulus, impact resistance, slidability, and the like, and therefore is widely used for electronic devices, vehicles, and the like as fibers, films, gears, bearings, and the like. Polyoxymethylene (POM) is inexpensive and versatile, and thus the demand is expanding.

Trioxane (1,3,5-trioxane, TOX), which is used as a raw material of polyoxymethylene (POM), is also in growing demand. Therefore, a method for producing trioxane (TOX) in high yield is required.

For example, Patent Literature 1 describes an invention relating to a method for producing trioxane. The method for producing trioxane includes 1) a step of bringing formaldehyde into contact with a catalyst containing at least methanesulfonic acid; 2) a step of performing a reaction to trimerize the formaldehyde to trioxane; and 3) a step of separating trioxane from a reaction medium.

Patent Literature 1 describes that the yield of trioxane and the selection rate of trioxane can be improved according to the method for producing trioxane.

Citation List

Patent Literature

Patent Literature 1: JP 2017-523998 A

Summary of Invention

Technical Problem

According to the method described in Patent Literature 1, trioxane can be produced in high yield. However, it has been found that continuous production may be difficult because methanesulfonic acid is used.

Under such circumstances, a means capable of continuously producing trioxane in high yield is required.

Solution to Problem

The present invention includes, for example, the following aspects.

[1] A composition containing:

(A) 58.5 to 65.5 mass% of formaldehyde;
(B) 0.7 to 4.5 mass% of methanesulfonic acid; and
(C) less than 0.007 mass% of a metal salt,

wherein a total content of the formaldehyde (A) and the methanesulfonic acid (B) is less than 68 mass%, and the metal salt (C) contains at least one selected from a group consisting of a nitrate, a nitrite, and a persulfate.

[2] The composition according to [1], wherein a content of the formaldehyde (A) is 60 to 65.5 mass%.

[3] The composition according to [1] or [2], wherein a content of the methanesulfonic acid (B) is 0.7 to 3.5 mass%.

[4] The composition according to any one of [1] to [3], wherein a content of the metal salt (C) is 0.002 to 0.004 mass%.

[5] A method for producing trioxane, including a step of obtaining trioxane by causing a reaction of a solution containing formaldehyde (A), methanesulfonic acid (B), and a metal salt (C),

wherein a reaction liquid at the time of equilibrium in the step of obtaining trioxane is the composition according to any one of [1] to [4].

Advantageous Effects of Invention

According to the present invention, a means capable of continuously producing trioxane in high yield is provided.

Description of Embodiments

Hereinafter, embodiments for carrying out the present invention will be described in detail.

<Composition>

The composition according to the present invention contains 58.5 to 65.5 mass% of formaldehyde (A), 0.7 to 4.5 mass% of methanesulfonic acid (B), and less than 0.007 mass% of a metal salt (C). At this time, the total content of the formaldehyde (A) and the methanesulfonic acid (B) is less than 68 mass%. The metal salt (C) contains at least one selected from the group consisting of a nitrate, a nitrite, and a persulfate.

According to the above composition, trioxane can be continuously produced in high yield.

Specifically, trioxane can be produced in high yield using the methanesulfonic acid (B) as an acid catalyst.

On the other hand, continuous production may be difficult due to the use of the methanesulfonic acid (B). Specifically, since the methanesulfonic acid (B) has metal corrosive properties, there is a case where the methanesulfonic acid (B) corrodes equipment (for example, a pipe) made of a metal (for example, made of stainless steel (SUS)) used in continuous production (especially industrial production or production at large scale). As a result, continuous production may be hindered. Since methanesulfonic acid is a strong acid, the formaldehyde (A) may be polymerized and converted into paraformaldehyde or the like in the presence of the methanesulfonic acid (B). Paraformaldehyde or the like may adhere to a pipe or the like, deposit, and block the pipe or the like, which may hinder continuous production.

However, according to the composition of the present invention, trioxane can be continuously produced. Specifically, use of a predetermined metal salt (C) forms a protective film on the surface of equipment made of a metal, and can prevent corrosion of the equipment made of a metal. In addition, control of the contents of the formaldehyde (A), the methanesulfonic acid (B), and the metal salt (C) in the composition within predetermined ranges can prevent the formation of paraformaldehyde or the like. As a result, corrosion of equipment made of a metal and blocking of a pipe or the like can be prevented during continuous production, and continuous production can be suitably performed.

Trioxane is formed by a trimerization reaction of formaldehyde, and at this time, the reaction is an equilibrium reaction. Here, the composition according to the present invention means a reaction liquid at the time of equilibrium of the reaction. When the reaction liquid at the time of equilibrium has the formulation of the composition of the present invention, trioxane can be continuously produced in high yield, and corrosion of equipment made of a metal and blocking of a pipe or the like can be prevented during continuous production.

In the present specification, the "time of equilibrium" means a time point at which no change is observed in the formulation of the reaction liquid after conditions are fixed. For example, in the continuous production of trioxane, when the formaldehyde (A) is additionally added at a single time, the content of the formaldehyde (A) in the reaction liquid increases at the time of addition. In this case, as formaldehyde is converted into trioxane, the content of formaldehyde gradually decreases, and the content of formaldehyde becomes a constant value at a certain time point. The time point at which such equilibrium is reached is the time of equilibrium, and the reaction liquid at the time of equilibrium is the composition of the present invention. In addition, for example, when a certain amount of trioxane to be formed is continuously distilled off, trioxane to be formed is sequentially distilled off, so that the content of trioxane in the reaction liquid gradually decreases, and the reaction liquid shifts to a new equilibrium condition on the premise of continuous distillation of trioxane. In this case, the content of formaldehyde becomes a constant value at a certain time point. The time point at which the equilibrium is reached under such a new equilibrium condition is the time of equilibrium, and the reaction liquid at the time of equilibrium is the composition of the present invention. The formulation of the composition (reaction liquid) can be measured by the following method. That is, the content of the formaldehyde (A) can be measured by neutralization titration with an acid of sodium hydroxide generated by causing a reaction with sodium sulfite. The content of the methanesulfonic acid (B) and the content of the metal salt (C) can be measured by ion chromatography.

The formulation of the composition according to the present invention (the formulation of the reaction liquid at the time of equilibrium) can be adjusted by, for example, changing the reaction conditions, adding the formaldehyde (A), adding the methanesulfonic acid (B), adding the metal salt (C), distilling off trioxane to be formed, or the like.

[Formaldehyde (A)]

Formaldehyde is trimerized by the action of an acid to form trioxane.

The content of formaldehyde with respect to the total mass of the composition is 58.5 to 65.5 mass%, preferably 60 to 65.5 mass%, and is more preferably 62 to 65.5 mass%, and still more preferably 62 to 65 mass% from the viewpoint of achieving a higher yield of trioxane. The content of formaldehyde is preferably 58.5 mass% or more, because trioxane can be produced in high yield. Meanwhile, the content of formaldehyde is preferably 65.5 mass% or less because blocking of a pipe or the like due to formation of paraformaldehyde or the like can be prevented.

Since formaldehyde is a gas, examples of a raw material used for producing the composition include an aqueous formaldehyde solution (formalin), paraformaldehyde (formaldehyde oligomer), and polyoxymethylene (formaldehyde polymer). Since the composition is preferably a solution containing water and/or an organic solvent as described later, formaldehyde is present in a dissolved form in water and/or an organic solvent in the composition.

[Methanesulfonic acid (B)]

Methanesulfonic acid promotes the conversion of formaldehyde into trioxane.

The content of methanesulfonic acid with respect to the total mass of the composition is 0.7 to 4.5 mass%, and is preferably 0.7 to 3.5 mass% from the viewpoint of achieving a higher yield of trioxane, and is more preferably 0.7 to 2.5 mass% from the viewpoint of being able to prevent blocking of a pipe or the like due to formation of paraformaldehyde or the like. The content of methanesulfonic acid is preferably 0.7 mass% or more because trioxane can be produced in high yield. Meanwhile, the content of methanesulfonic acid is preferably 4.5 mass% or less because blocking of a pipe or the like due to formation of paraformaldehyde or the like can be prevented.

The total content of the formaldehyde (A) and the methanesulfonic acid (B) with respect to the total mass of the composition is less than 68 mass%, preferably 50 mass% or more and less than 68 mass%, more preferably 58 to 67.5 mass%, and still more preferably 60 to 66.5 mass%. Meanwhile, the total content is preferably less than 68 mass% because blocking of a pipe or the like due to formation of paraformaldehyde or the like can be prevented.

The mass ratio of the content of the formaldehyde (A) to the total content of the formaldehyde (A) and the methanesulfonic acid (B) (A/(A+B)) is preferably 0.900 to 0.995, more preferably 0.930 to 0.995, still more preferably 0.950 to 0.990, even more preferably 0.955 to 0.990, and still even more preferably 0.970 to 0.985. The mass ratio is preferably in the above range because trioxane can be produced in high yield, and blocking of a pipe or the like due to formation of paraformaldehyde or the like can be prevented.

[Another acid]

The composition according to the present invention may contain another acid. Here, the "another acid" means an acid other than the methanesulfonic acid (B).

The another acid is not particularly limited, and examples thereof include hydrochloric acid, sulfuric acid, nitric acid, phosphoric acid, perchloric acid, p-toluenesulfonic acid, trifluoromethanesulfonic acid, benzenesulfonic acid, toluenesulfonic acid, and 1,5-naphthalenedisulfonic acid. These other acids may be used alone or two or more types thereof may be used in combination.

The content of the another acid with respect to the total mass of the composition is preferably 0.1 to 10 mass%, more preferably 0.1 to 3 mass%, and still more preferably 0.3 to 1 mass%. When two or more types of other acids are contained, the total content thereof is preferably in the above range.

[Metal salt (C)]

The metal salt forms a protective film on a metal surface of a pipe or the like to prevent metal corrosion caused by methanesulfonic acid. In the present description, the "metal" means steel and a nonferrous metal, and is preferably stainless steel (SUS) and carbon steel, and more preferably stainless steel (SUS).

The metal salt contains at least one selected from the group consisting of a nitrate, a nitrite, and a persulfate.

The nitrate is not particularly limited, and examples thereof include sodium nitrate, potassium nitrate, magnesium nitrate, cerium nitrate, cerium ammonium nitrate, iron nitrate, molybdenum nitrate, and vanadium nitrate.

The nitrite is not particularly limited, and examples thereof include sodium nitrite, potassium nitrite, magnesium nitrite, cerium nitrite, cerium ammonium nitrite, iron nitrite, molybdenum nitrite, and vanadium nitrite.

The persulfate is not particularly limited, and examples thereof include sodium persulfate, potassium persulfate, magnesium persulfate, cerium persulfate, cerium ammonium persulfate, iron persulfate, molybdenum persulfate, and vanadium persulfate.

Among these, the metal salt preferably contains at least one selected from the group consisting of a nitrate and a nitrite, more preferably contains a nitrate, still more preferably contains at least one selected from the group consisting of sodium nitrate, potassium nitrate, magnesium nitrate, cerium nitrate, and ammonium cerium nitrate, and particularly preferably contains sodium nitrate and/or potassium nitrate. The metal salts may be used alone or two or more types thereof may be used in combination.

The content of the metal salt with respect to the total mass of the composition is less than 0.007 mass%, preferably 0.0001 to 0.0065 mass%, and more preferably 0.001 to 0.006 mass%, and is further preferably 0.002 to 0.004 mass% from the viewpoint of being able to prevent blocking of a pipe or the like due to formation of paraformaldehyde or the like. The content of the metal salt is preferably less than 0.007 mass% because blocking of a pipe or the like due to formation of paraformaldehyde or the like can be prevented.

[Water]

The composition according to the present invention preferably contains water. When the composition contains water, the formaldehyde (A) can be dissolved. This can prevent blocking of a pipe or the like due to formation of paraformaldehyde or the like. When the composition contains water, trioxane and water form an azeotropic mixture (trioxane : water = 70 : 30 (mass ratio), azeotropic point: 91.3°C), and trioxane can be distilled off from the reaction system. This can bias the equilibrium reaction to convert formaldehyde to trioxane to the side where trioxane is formed.
The content of water with respect to the total mass of the composition is preferably 20 to 40 mass%, more preferably 30 to 40 mass%, and still more preferably 35 to 40 mass%. The content of water is preferably 20 mass% or more because formaldehyde can be suitably dissolved, and blocking of a pipe or the like due to formation of paraformaldehyde or the like can be prevented. Meanwhile the content of water is preferably 40 mass% or less because trioxane can be produced in high yield.

[Organic Solvent]

The composition according to the present invention may contain an organic solvent. The organic solvent can prevent formation of paraformaldehyde or the like by suitably dissolving formaldehyde.

[Trioxane]

The composition according to the present invention is a reaction liquid at the time of equilibrium, and therefore usually contains trioxane.
The content of trioxane with respect to the total mass of the composition is preferably 20 mass% or less, more preferably 10 mass% or less, and still more preferably 5 mass% or less.

[Additive]

The composition according to the present invention may contain an additive. The additive is not particularly limited, and examples thereof include an antifoaming agent, a surfactant, and a stabilizer. These additives may be used alone or two or more types thereof may be used in combination.

[Combination of contents]

Preferred contents of the formaldehyde (A), the methanesulfonic acid (B), and the metal salt (C) contained in the composition are as described above, and can be each appropriately combined.
In an embodiment, from the viewpoint of suitably obtaining the respective effects of production of trioxane in high yield, prevention of blocking of a pipe or the like due to formation of paraformaldehyde or the like, and prevention of metal corrosion, a preferable combination of the contents of (A) to (C) in the composition is as follows: (A): 59 to 65 mass%, (B): 0.5 to 2.5 mass%, and (C): 0.002 to 0.004 mass%, and the total content of the formaldehyde (A) and the methanesulfonic acid (B): 60 to 66 mass%, and a more preferable combination of the contents of (A) to (C) in the composition is as follows: (A): 60 to 64 mass%, (B): 1 to 2 mass%, and (C): 0.002 to 0.004 mass%, and the total content of the formaldehyde (A) and the methanesulfonic acid (B): 61 to 66 mass%. In another embodiment, from the viewpoint of suitably obtaining trioxane in high yield, a preferable combination of the contents of (A) to (C) in the composition is as follows: (A): 62 to 65 mass%, (B): 0.5 to 3.5 mass%, and (C): 0.002 to 0.004 mass%, and the total content of the formaldehyde (A) and the methanesulfonic acid (B): 64 to 67 mass%, and a more preferable combination of the contents of (A) to (C) in the composition is as follows: (A): 63.5 to 65 mass%, (B): 1 to 3 mass%, and (C): 0.002 to 0.004 mass%, and the total content of the formaldehyde (A) and the methanesulfonic acid (B): 65 to 67 mass%.

<Method for producing trioxane>

According to an embodiment of the present invention, a method for producing trioxane is provided. A method for producing the trioxane includes a step of obtaining trioxane by causing a reaction of a solution containing formaldehyde (A), methanesulfonic acid (B), and a metal salt (C). At this time, the reaction liquid at the time of equilibrium in the step of obtaining trioxane is the composition described above. The method for producing trioxane may further include a trioxane purification step.

[Trioxane production step]

A trioxane production step is a step of obtaining trioxane by causing a reaction of a solution containing formaldehyde (A), methanesulfonic acid (B), and a metal salt (C).

(Solution)

The solution contains formaldehyde (A), methanesulfonic acid (B), and a metal salt (C). In addition, another acid, water, an organic solvent, an additive, or the like may be further contained.

As the formaldehyde (A), the methanesulfonic acid (B), the metal salt (C), another acid, water, an organic solvent, an additive, or the like, those described above are used.

The contents of the formaldehyde (A), the methanesulfonic acid (B), and the metal salt (C) in the solution are preferably such amounts that the reaction liquid at the time of equilibrium becomes the above-described composition in consideration of the production conditions set in the trioxane production step. In addition, the addition amount of another acid, water, an organic solvent, an additive, or the like can be appropriately set.

(Reaction)

The formaldehyde (A) is trimerized in the presence of the methanesulfonic acid (B) to form trioxane. At this time, the trimerization is an equilibrium reaction.

Formic acid, glycolic acid ($CH_2OHCOOH$), methanol, or carbon dioxide may be formed by a side reaction. These byproducts can also react to form an additional byproduct. However, when the reaction liquid at the time of equilibrium is adjusted to become the composition according to the present invention, trimerization of formaldehyde can occur more selectively, and thus trioxane can be produced in high yield.

The reaction method is not particularly limited, and a known method is applied. Examples of the reaction method include a method of heating the solution.

The reaction temperature is not particularly limited, and is preferably 70 to 150°C, more preferably 80 to 120°C, and particularly preferably 90 to 120°C. By adjusting the reaction temperature, trioxane to be formed can be distilled off to the outside of the reaction system, and trioxane can be obtained in higher yield.

The reaction pressure is not particularly limited, but is preferably $0.1 \times 10^5$ to $10 \times 10^5$ Pa, more preferably $1 \times 10^5$ to $5 \times 10^5$ Pa, and still more preferably $1 \times 10^5$ to $2 \times 10^5$ Pa. By adjusting the reaction pressure, trioxane to be formed can be distilled off to the outside of the reaction system, and trioxane can be obtained in higher yield.

The reaction time is not particularly limited, but is preferably 0.1 to 10 hours, more preferably 0.3 to 5 hours, and still more preferably 0.5 to 4 hours. By adjusting the reaction time, trioxane can be efficiently produced.

Since trimerization of formaldehyde is an equilibrium reaction, it is preferable to distill off trioxane as a product from the reaction system in the reaction process. For example, when the composition contains water, trioxane becomes an azeotropic mixture with water, and is distilled off to the outside of the reaction system under predetermined reaction conditions.

The distilled trioxane (for example, an azeotropic mixture of trioxane and water) is preferably purified as it is by a trioxane purification step described later.

When trioxane is continuously produced, it can be carried out by sequentially adding an aqueous formaldehyde solution into a reaction system in which methanesulfonic acid and a metal salt are present, and sequentially distilling off the obtained trioxane to the outside of the reaction system. At this time, the aqueous formaldehyde solution to be added is preferably added in such an amount that the reaction liquid at the time of equilibrium has the formulation of the composition according to the present invention in consideration of the reaction conditions, the amount of trioxane distilled off to the outside of the reaction system, and the like.

[Trioxane purification step]

The trioxane purification step is a step of purifying trioxane obtained in the trioxane production step. Here, examples of the "obtained trioxane" include trioxane (preferably, an azeotropic mixture of trioxane and water) distilled off to the outside of the reaction system during the reaction, and a reaction solution after the reaction. At this time, the "obtained trioxane" can contain, in addition to trioxane, formaldehyde, water, an organic solvent, an additive, a byproduct (formic acid, glycolic acid ($CH_2OHCOOH$), methanol, or carbon dioxide), a component derived from splash generated in the reaction system, a component derived from a pipe, or the like.

The purification method is not particularly limited, and examples thereof include distillation, crystallization, filtration, and extraction. Among these, the purification method preferably includes distillation and/or extraction, and more preferably includes distillation. As the purification method, a known method can be appropriately adopted.

It is preferable to reuse impurities other than trioxane separated in the purification step, for example, at least one of formaldehyde, water, an organic solvent, and the like in the trioxane production step.

Examples

Hereinafter, the present invention will be described in more detail with reference to Examples, but the present invention is not limited to these Examples.

[Example 1]

(Production of trioxane)

A solution containing formaldehyde (A), methanesulfonic acid (B), sodium nitrate (C), and water was charged into a reactor (having a configuration of reactor-pipe (distillation line)-Allihn condenser-distillate storage flask-pipe (return line)-the reactor) in which a distillate storage flask and an Allihn condenser are connected through two pipes (a distillation line and a return line), and heated at a heat medium temperature of 130°C for 3 hours to produce trioxane. In the reaction liquid (composition) at the time of equilibrium after 3 hours, the content of the formaldehyde (A) was measured by neutralization titration with an acid of sodium hydroxide generated by causing a reaction with sodium sulfite after methanesulfonic acid and an acid component (such as formic acid) as a byproduct in the composition was neutralized with dilute hydrochloric acid in advance. The content of the methanesulfonic acid (B) and the content of the metal salt (C) in the reaction liquid (composition) at the time of equilibrium were measured by ion chromatography (device name: Dionex ICS-2100, column: Dionex IonPac AS11-HC and Dionex IonPac AG11-HC). As a result, with respect to the total mass of the composition, the content of (A) was 60 mass%, the content of (B) was 1 mass%, and the content of (C) was 0.003 mass%.

At this time, in the reaction, the produced trioxane formed an azeotropic mixture with water and was distilled off from the reaction system. This biases the equilibrium reaction to form trioxane from formaldehyde to the side where trioxane is formed. The azeotropic mixture of trioxane and water was cooled and liquefied in an Allihn condenser through a pipe (distillation line) connected to the reactor and stored as a trioxane distillate in a distillate storage flask connected to the Allihn condenser. When the storage amount of the distillate storage flask was exceeded, the trioxane distillate in the distillate storage flask was returned to the reactor through a pipe (return line).

[Example 2]

A composition was produced in the same manner as in Example 1 except that the content of (A) was adjusted to 64 mass% in the reaction liquid (composition) at the time of equilibrium.

[Example 3]

A composition was produced in the same manner as in Example 1 except that the content of (A) was adjusted to 64 mass%, and the content of (B) was adjusted to 2 mass% in the reaction liquid (composition) at the time of equilibrium.

[Example 4]

A composition was produced in the same manner as in Example 1 except that the content of (B) was adjusted to 3 mass% in the reaction liquid (composition) at the time of equilibrium.

[Example 5]

A composition was produced in the same manner as in Example 1 except that the content of (A) was adjusted to 64 mass%, and the content of (B) was adjusted to 3 mass% in the reaction liquid (composition) at the time of equilibrium.

[Example 6]

A composition was produced in the same manner as in Example 1 except that the content of (A) was adjusted to 63 mass%, and the content of (B) was adjusted to 4 mass% in the reaction liquid (composition) at the time of equilibrium.

[Example 7]

A composition was produced in the same manner as in Example 1 except that the content of (A) was adjusted to 64 mass%, the content of (B) was adjusted to 2 mass%, and the content of (C) was adjusted to 0.005 mass% in the reaction liquid (composition) at the time of equilibrium.

[Example 8]

A composition was produced in the same manner as in Example 1 except that the content of (A) was adjusted to 63 mass%, the content of (B) was adjusted to 4 mass%, and the content of (C) was adjusted to 0.005 mass% in the reaction liquid (composition) at the time of equilibrium.

[Example 9]

A composition was produced in the same manner as in Example 1 except that the content of (A) was adjusted to 64 mass%, the content of (B) was adjusted to 2 mass%, and the content of (C) was adjusted to 0.001 mass% in the reaction liquid (composition) at the time of equilibrium.

[Comparative Example 1]

A composition was produced in the same manner as in Example 1 except that the content of (B) was adjusted to 0.5 mass% in the reaction liquid (composition) at the time of equilibrium.

[Comparative Example 2]

A composition was produced in the same manner as in Example 1 except that the content of (A) was adjusted to 64 mass%, and the content of (B) was adjusted to 0.5 mass% in the reaction liquid (composition) at the time of equilibrium.

[Comparative Example 3]

A composition was produced in the same manner as in Example 1 except that the content of (B) was adjusted to 5 mass% in the reaction liquid (composition) at the time of equilibrium.

[Comparative Example 4]

A composition was produced in the same manner as in Example 1 except that the content of (A) was adjusted to 66 mass% in the reaction liquid (composition) at the time of equilibrium.

[Comparative Example 5]

A composition was produced in the same manner as in Example 1 except that the content of (A) was adjusted to 64 mass%, and the content of (B) was adjusted to 4 mass% in the reaction liquid (composition) at the time of equilibrium.

[Comparative Example 6]

A composition was produced in the same manner as in Example 1 except that the content of (A) was adjusted to 58 mass%, and the content of (B) was adjusted to 2 mass% in the reaction liquid (composition) at the time of equilibrium.

[Comparative Example 7]

A composition was produced in the same manner as in Example 1 except that the content of (B) was adjusted to 2 mass%, and the content of (C) was adjusted to 0.01 mass% in the reaction liquid (composition) at the time of equilibrium.

[Comparative Example 8]

A composition was produced in the same manner as in Example 1 except that the content of (A) was adjusted to 64 mass%, the content of (B) was adjusted to 2 mass%, and the content of (C) was adjusted to 0.01 mass% in the reaction liquid (composition) at the time of equilibrium.

[Comparative Example 9]

A composition was produced in the same manner as in Example 1 except that the content of (B) was adjusted to 4 mass%, and the content of (C) was adjusted to 0.01 mass% in the reaction liquid (composition) at the time of equilibrium.

[Comparative Example 10]

A composition was produced in the same manner as in Example 1 except that the content of (A) was adjusted to 64 mass%, and the content of (B) was adjusted to 2 mass% in the reaction liquid (composition) at the time of equilibrium, and (C) was not added.

[Comparative Example 11]

A composition was produced in the same manner as in Example 1 except that the content of (A) was adjusted to 63 mass%, and the content of (B) was adjusted to 4 mass% in the reaction liquid (composition) at the time of equilibrium, and (C) was not added.

The formulations of the compositions in Examples 1 to 9 and Comparative Examples 1 to 11 are shown in Table 1 below.

**[Table 1]**

|  | A (mass%) | B (mass%) | C (mass%) | A + B (mass%) |
|---|---|---|---|---|
| Example 1 | 60 | 1 | 0.003 | 61 |
| Example 2 | 64 | 1 | 0.003 | 65 |
| Example 3 | 64 | 2 | 0.003 | 66 |
| Example 4 | 60 | 3 | 0.003 | 63 |
| Example 5 | 64 | 3 | 0.003 | 67 |
| Example 6 | 63 | 4 | 0.003 | 67 |
| Example 7 | 64 | 2 | 0.005 | 66 |
| Example 8 | 63 | 4 | 0.005 | 67 |
| Example 9 | 64 | 2 | 0.001 | 66 |
| Comparative Example 1 | 60 | 0.5 | 0.003 | 60.5 |
| Comparative Example 2 | 64 | 0.5 | 0.003 | 64.5 |
| Comparative Example 3 | 60 | 5 | 0.003 | 65 |
| Comparative Example 4 | 66 | 1 | 0.003 | 67 |
| Comparative Example 5 | 64 | 4 | 0.003 | 68 |
| Comparative Example 6 | 58 | 2 | 0.003 | 60 |
| Comparative Example 7 | 60 | 2 | 0.01 | 62 |
| Comparative Example 8 | 64 | 2 | 0.01 | 66 |
| Comparative Example 9 | 60 | 4 | 0.01 | 64 |
| Comparative Example 10 | 64 | 2 | 0 | 66 |
| Comparative Example 11 | 63 | 4 | 0 | 67 |

[Evaluation]

The compositions or the trioxane distillates in the distillate storage flasks of Examples 1 to 9 and Comparative Examples 1 to 11 were subjected to various evaluations.

# EP 4 534 521 A1

(Trioxane content ratio)

The trioxane distillate was recovered from the distillate storage flask after the reaction equilibrium was reached (after 3 hours), the content of trioxane contained in the trioxane distillate was measured by gas chromatography, and the trioxane content ratio (TOX content ratio) with respect to the total mass of the trioxane distillate was calculated. At this time, in the measurement by gas chromatography, Nexis GC-2030 (manufactured by Shimadzu Corporation) was used as an apparatus, and DB-WAX (manufactured by Agilent Technology) was used as a column. A case where the TOX content ratio is high indicates that trioxane could be produced in high yield. The obtained results are shown in Table 2 below.

(Appearance of reaction liquid)

The reaction liquid (composition) in the reactor after the reaction equilibrium was reached (after 3 hours) was visually observed, and the appearance of the reaction liquid was evaluated according to the following criteria. A case where the evaluation result of the appearance of the reaction liquid is high indicates that paraformaldehyde or the like was not formed from formaldehyde in the process of producing trioxane. Therefore, when trioxane is produced using the composition, blocking of a pipe or the like hardly occurs, and thus it can be said that the composition is suitable for continuous production. The obtained results are shown in Table 2 below.
○: Colorless and transparent
△: Slightly cloudy
✕: Markedly cloudy

(Stainless steel (SUS) corrosive properties)

A test piece (length: 60 mm, width: 20 mm, thickness: 2 mm, weld line made of same material on both surfaces, including φ 4 mm drill through hole) of SUS316L was immersed in 100 g of the reaction liquid (composition) in the reactor after the reaction equilibrium was reached (after 3 hours), and allowed to stand at 90°C for 1 day. The mass reduction rate (mass) of the test piece after immersion was calculated by the following formula.

$$\text{Mass reduction rate (mass\%)} = \frac{\text{Mass of test piece before immersion - Mass of test piece after immersion}}{\text{Mass of test piece before immersion}} \times 100$$

SUS corrosive properties were evaluated according to the following criteria. A case where the evaluation result of the SUS corrosive properties is high indicates that the composition hardly corrodes equipment made of SUS. The obtained results are shown in Table 2 below.
○: The mass reduction rate is less than 0.2 mass%
✕: The mass reduction rate is 0.2 mass% or more.

[Table 2]

|  |  | TOX content ratio* | Appearance of reaction liquid | SUS corrosive properties |
|---|---|---|---|---|
|  | Example 1 | 17.2 | ○ | ○ |
|  | Example 2 | 19.4 | ○ | ○ |
|  | Example 3 | 19.5 | ○ | ○ |
|  | Example 4 | 18 | △ | ○ |
|  | Example 5 | 19.5 | △ | ○ |
|  | Example 6 | 18.8 | △ | ○ |
|  | Example 7 | 19.7 | △ | ○ |
|  | Example 8 | 18.7 | △ | ○ |
|  | Example 9 | 19.5 | △ | ○ |

(continued)

|  | TOX content ratio* | Appearance of reaction liquid | SUS corrosive properties |
|---|---|---|---|
| Comparative Example 1 | 12 | ○ | ○ |
| Comparative Example 2 | 15.3 | ○ | ○ |
| Comparative Example 3 | - | × | ○ |
| Comparative Example 4 | - | × | ○ |
| Comparative Example 5 | - | × | ○ |
| Comparative Example 6 | 15.4 | ○ | ○ |
| Comparative Example 7 | 17 | × | ○ |
| Comparative Example 8 | 19.4 | × | ○ |
| Comparative Example 9 | 17.3 | × | ○ |
| Comparative Example 10 | 19.4 | ○ | × |
| Comparative Example 11 | 19 | △ | × |
| *-: Production was discontinued due to blocking of the pipe (return line). | | | |

From the results in Table 2, it is found that the compositions of Examples 1 to 9 enabled the production of trioxane in high yield, can prevent byproduction of paraformaldehyde or the like, and prevent corrosion of SUS. Therefore, it can be said that the compositions of Examples 1 to 9 enable the production of trioxane in high yield and are suitable for continuous production.

**Claims**

1.  A composition comprising:

    (A) 58.5 to 65.5 mass% of formaldehyde;
    (B) 0.7 to 4.5 mass% of methanesulfonic acid; and
    (C) less than 0.007 mass% of a metal salt,
    wherein a total content of the formaldehyde (A) and the methanesulfonic acid (B) is less than 68 mass%, and the metal salt (C) contains at least one selected from a group consisting of a nitrate, a nitrite, and a persulfate.

2.  The composition according to claim 1, wherein a content of the formaldehyde (A) is 60 to 65.5 mass%.

3.  The composition according to claim 1 or 2, wherein a content of the methanesulfonic acid (B) is 0.7 to 3.5 mass%.

4.  The composition according to any one of claims 1 to 3, wherein a content of the metal salt (C) is 0.002 to 0.004 mass%.

5.  A method for producing trioxane, comprising a step of obtaining trioxane by causing a reaction of a solution containing formaldehyde (A), methanesulfonic acid (B), and a metal salt (C),
    wherein a reaction liquid at the time of equilibrium in the step of obtaining trioxane is the composition according to any one of claims 1 to 4.

## INTERNATIONAL SEARCH REPORT

| International application No. |
|---|
| **PCT/JP2023/020399** |

**A. CLASSIFICATION OF SUBJECT MATTER**

*C07C 47/04*(2006.01)i; *C07C 309/04*(2006.01)i; *C07D 323/06*(2006.01)i
FI: C07C47/04; C07D323/06; C07C309/04

According to International Patent Classification (IPC) or to both national classification and IPC

**B. FIELDS SEARCHED**

Minimum documentation searched (classification system followed by classification symbols)

C07C47/04; C07C309/04; C07D323/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 40-008545 B1 (BADISCHE ANILIN- & SODA-FABRIK AG) 04 May 1965 (1965-05-04) example 3, p. 1, right column, line 36 to p. 2, left column, line 2 | 1-4 |
| A | | 5 |
| Y | JP 2017-523998 A (ARKEMA FRANCE) 24 August 2017 (2017-08-24) claim 1, paragraph [0026], examples | 1-4 |
| A | | 5 |
| Y | JP 11-241191 A (ELF ATOCHEM SA) 07 September 1999 (1999-09-07) claims 1-3 | 1-4 |
| A | | 5 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 August 2023** | **22 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/020399**

| Patent document cited in search report | | | Publication date (day/month/year) | Patent family member(s) | Publication date (day/month/year) |
|---|---|---|---|---|---|
| JP | 40-008545 | B1 | 04 May 1965 | (Family: none) | |
| JP | 2017-523998 | A | 24 August 2017 | US 2017/0233366 A1 claim 1, paragraph [0023], examples WO 2016/020592 A1 EP 3177602 A1 CN 106536499 A KR 10-2017-0024113 A | |
| JP | 11-241191 | A | 07 September 1999 | US 6120619 A claims 1-3 EP 931854 A1 KR 10-1999-0066898 A | |

Form PCT/ISA/210 (patent family annex) (January 2015)

**EP 4 534 521 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017523998 A